Europäisches Patentamt

European Patent Office

Office européen des brevets

(19)

(11) EP 1 271 152 A1

## (12) EUROPEAN PATENT APPLICATION
published in accordance with Art. 158(3) EPC

(43) Date of publication:
02.01.2003 Bulletin 2003/01

(51) Int Cl.7: **G01N 33/574**, G01N 33/53, C07K 7/08

(21) Application number: 01917677.5

(22) Date of filing: 30.03.2001

(86) International application number:
PCT/JP01/02723

(87) International publication number:
WO 01/075449 (11.10.2001 Gazette 2001/41)

(84) Designated Contracting States:
AT BE CH CY DE DK ES FI FR GB GR IE IT LI LU
MC NL PT SE TR

(30) Priority: 30.03.2000 JP 2000093569

(71) Applicant: Nippon Kayaku Kabushiki Kaisha
Tokyo 102-8172 (JP)

(72) Inventor: SEINO, Yuko
Omiya-shi, Saitama 330-0033 (JP)

(74) Representative:
Gille Hrabal Struck Neidlein Prop Roos
Patentanwälte,
Brucknerstrasse 20
40593 Düsseldorf (DE)

(54) **METHOD OF EXAMINING CANCER BY ASSAYING AUTOANTIBODY AGAINST MDM2 AND REAGENT THEREFOR**

(57) The present invention relates to a novel method for detecting a cancer by measuring an autoantibody to MDM2 in blood, a reagent therefor, and a kit therefor. For example, a MDM peptide fragment is used as an antigen, and an anti-MDM2 antibody produced by use of said antigen, or the labeled product thereof is used as an antibody for measuring the above autoantibody, to detect the cancer by an immunoassay.

Fig. 1

Measurement result of MDM2 autoantibody concentration by the competition assay

| Disease | Positive rate | Measurement value (%) |
|---|---|---|
| Normal subjct | | |
| Large intestinal cancer | 3/8(37.5%) | |
| Gastric cancer | 7/8(87.5%) | |
| Esophageal cancer | 4/4(100%) | |
| Lung cancer | 8/8(100%) | |
| Pancreatic cancer | 4/4(100%) | |
| Hepatoma | 5/5(100%) | |
| Gallbladder cancer | 2/2(100%) | |
| Prostatic cancer | 1/1(100%) | |
| Breast cancer | 4/8(50.0%) | |

0.0    5.0    10.0    15.0    20.0

(%)

EP 1 271 152 A1

**Description**

TECHNICAL FIELD

The present invention relates to a method for detecting a cancer by measuring an autoantibody to MDM2 existing in a test sample, and a reagent therefor.

BACKGROUND ART

MDM2(murine double minute-2) is a gene product existing locally in a cell nucleus as well as a histone, p53, a hormone receptor and the like, and it is known that MDM2 is amplified at a high frequency in a sarcoma. It is reported that autoantibodies to MDM2 exist in patients with various gynecological diseases (Cancer Letters, 96, 111-115(1995)).
Generally, it is known that a certain gene product existing locally in a cell nucleus is expressed excessively with malignant alteration of the cell compared with a normal cell. In a tumor-bearing host, it is known that the cancer cells are destroyed by the tumor immunoreaction and gene products existing in the cell nucleus are released outside the cell and that, as the result a humoral antibody is produced. Autoantibodies to a histone or p53 etc. are examples of such case (JP Laid-Open No.32766/1992, JP National publication of Japanese translation of PCT No.505719/1995 (WO93/21529), JP Laid-Open No.229933/1997 (UK Pat. No.2241782).
There is, however, a problem that a conventional measurement of autoantibody is too poor in specificity to cancer and it is difficult to discriminate between cancer patients and subjects without cancer.
An object of the present invention is to provide a simple and easy method for detecting a cancer by measuring an autoantibody, particularly, the method enabling diagnosis of an early stage of cancer; and a reagent and a kit therefore.

DISCLOSURE OF THE INVENTION

The present inventors have studied to attain the above object, found out that measurement of autoantibodies to MDM2 in blood of patients with various cancers is effective for the diagnosis of the cancer, and completed the present invention based on this finding.
Namely the present invention is as follows:

(1) Amethod for detecting a cancer characterized by measuring autoantibody to MDM2 existing in a test sample.
(2) The method according to the above (1), wherein said cancer is an early stage of cancer.
(3) The method according to the above (1), wherein said cancer is an epithelial cancer.
(4) The method according to the above (3), wherein said epithelial cancer is pulmonary squamous cell carcinoma, pulmonary adenocarcinoma or pulmonary small cell carcinoma.
(5) The method according to any one of the above (1) to (4),
wherein said measurement of the autoantibodies is carried out by an immunoassay.
(6) The method according to the above (5), wherein said immunoassay is a competition assay, an inhibition assay or a sandwich assay.
(7) The method according to the above (5), wherein said immunoassay utilizes a peptide fragment of MDM2 (hereinafter referred to as a MDM2 peptide fragment) as an antigen for the immunoassay.
(8) The method according to the above (5), wherein said immunoassay uses an anti-MDM2 antibody or the labeled product thereof.
(9) The method according to the above (7), wherein said MDM2 peptide fragment is an N-terminal region peptide fragment of MDM2 or the modified product thereof.
(10) The method according to the above (7) or (9), wherein said MDM2 peptide fragment is immobilized on a solid-phase.
(11) A peptide as defined by the following (a) or (b):

(a) a peptide which has the amino acid sequence as shown by SEQ No.1 in SEQUENCE LISTING and has a function to bind an autoantibody to MDM2; or
(b) a peptide which has an amino acid sequence obtained by the deletion, substitution or addition of amino acid(s) in a part of the amino acid sequence as shown by SEQ No.1 in SEQUENCE LISTING and has a function to bind an autoantibody to MDM2.

(12) A reagent for detecting a cancer comprising MDM2, a MDM2 peptide fragment or their labeled product(s).
(13) A reagent for detecting a cancer comprising an anti-MDM2 antibody or the labeled product thereof.

(14) The reagent for detecting a cancer according to the above (12), wherein said cancer is an epithelial cancer.

(15) The reagent for detecting a cancer according to the above (12), wherein said MDM2 peptide fragment is an N-terminal region peptide fragment of MDM2 or the modified product thereof.

(16) The reagent for detecting a cancer according to the above (12) or (15), wherein said MDM2 peptide fragment is immobilized on a solid-phase.

(17) The reagent for detecting a cancer according to the above (12), wherein said MDM2 peptide fragment is a peptide as defined by the following (a) or (b):

(a) a peptide which has the amino acid sequence as shown by SEQ No.1 in SEQUENCE LISTING and has a function to bind an autoantibody to MDM2; or

(b) a peptide which has an amino acid sequence obtained by the deletion, substitution or addition of amino acid(s) in a part of the amino acid sequence as shown by SEQ No.1 in SEQUENCE LISTING and has a function to bind an autoantibody to MDM2.

(18) A kit for detecting a cancer comprising the reagent for detecting a cancer according to any one of the above (12) to (17).

(19) The kit for detecting a cancer according to the above (18) further comprising a standard solution for preparing a calibration curve.

(20) Use of MDM2, a MDM2 peptide fragment or their labeled products for producing a reagent for detecting a cancer comprising said MDM2, said MDM2 peptide fragment or said labeled products thereof.

(21) The method for detecting a cancer according to claim 8, wherein said anti-MDM2 antibody is either an antibody produced by using a MDM2 peptide fragment as an antigen, or the modified product thereof.

BRIEF DESCRIPTION OF DRAWINGS

Fig.1 shows a measurement result on an autoantibody to MDM2 in blood by a competition assay in Example 3.

Fig.2 shows a calibration curve on a rabbit polyclonal anti-MDM2 antibody.

Fig.3 shows a measurement result on an autoantibody to MDM2 in blood by a competition assay in Example 4.

Fig.4 shows a measurement result on an autoantibody to MDM2 in blood by stage in breast cancer.

Fig.5 shows a measurement result on an autoantibody to MDM2 in blood by stage in gastric cancer.

Fig.6 shows a measurement result on an autoantibody to MDM2 in blood by histological type in lung cancer.

Fig.7 shows a measurement result on an autoantibody to MDM2 in blood by differentiation level in pulmonary squamous cell carcinoma and pulmonary adenocarcinoma.

Fig.8 shows a measurement result on an autoantibody to MDM2 in blood by differentiation level in gastric cancer .

BEST MODE FOR CARRYING OUT THE INVENTION

The present invention relates to the method for detecting a cancer by measurement of an autoantibody to MDM2 existing in a test sample. The cancer for detecting may be an epithelial cancer or not, but is preferably an epithelial cancer such as gastrointestinal cancer (gastric cancer, esophageal cancer, large intestinal cancer, hepatoma, pancreatic cancer, gallbladder cancer), lung cancer and breast cancer.

The sample used in the present invention is not limited to any particular one as long as it is body fluid containing the above autoantibody, but includes preferably blood, serum or plasm, and more preferably human blood, serum or plasm.

MDM2 is a gene product existing locally in a cell, and transcriptional activity of the MDM2 gene is accelerated by p53, one of tumor suppressor gene products. It is also known that MDM2 binds p53 and suppresses the transcriptional activity of p53. Recently, with respect to suppression mechanism of activity of p53 by MDM2, it has been found that MDM2 acts as a ubiquitin ligase and ubiquitinates p53 to decompose. Accordingly, MDM2 is considered to play an important role in the carcinogenesis mechanism of a cell because it is observed that MDM2 is highly expressed in various cancer cells and MDM2 acts as the decomposition enzyme to decompose p53.

An autoantibody to MDM2 is an antibody that an own antibody-producing cell produces to MDM2 existing in its own body and it has property to bind MDM2 specifically.

A method for measuring an autoantibody to MDM2 in blood is not limited to any particular one, but an immunoassay is suitable for the method. For example, MDM2 or a peptide fragment of MDM2 (hereinafter simply referred to as a MDM2 peptide fragment)is used as an antigen to immunize an animal to get an antibody, which can be used for the measurement method. To use a MDM2 peptide fragment as an antigen is more preferable, because the peptide fragment is stable and easily available, as will be described hereinafter.

An animal species to immunize to produce the antibody is not limited to any particular one, but the preferable example includes rabbits, goats, mice, rats, horses, pigs, cows and chickens and the like. The antibody to use in the present invention may be a polyclonal antibody or amonoclonal antibody.

In the present invention, the term, anti-MDM2 antibody, means what is obtained by using MDM2 and/or a MDM2 peptide fragment as the antigen, unless otherwise stated. The term "anti-MDM2 antibody" is also used for a modified product of an anti-MDM2 antibody such as a biotinylated antibody, that it does not included in a labeled product. These antibodies may be used in the form of a labeled antibody. A preferable anti-MDM2 antibody includes an antibody produced by using a MDM2 peptide fragment as the antigen, and the modified product (such as the biotinylated anti-MDM2 antibody).

An assay using the antibody includes a competition assay, an inhibition assay and a sandwich assay. MDM2 and a MDM2 peptide fragment to use for an assay in the present invention include their modified products obtained by modifying a part of amino acids by the biotinylation for example.

The competition assay can be carried out by reacting both the above anti-MDM2 antibody labeled with a marker and an autoantibody in a sample competitively with MDM2 or a MDM2 peptide fragment, and then determining an amount of the bound anti-MDM2 antibody, from which the amount of the autoantibody in the sample is calculated. Alternatively, it can be carried out by reacting both the non-labeled anti-MDM2 antibody and an autoantibody in a sample competitively with MDM2 or a MDM2 peptide fragment, and then reacting with a marker-labeled antibody to the anti-MDM2 antibody or a marker-labeled avidin, an avidin analogue or a derivative thereof, following by determining an amount of the anti-MDM2 antibody bound to the MDM2 or the MDM2 peptide fragment by using the marker.

The inhibition assay can be carried out by reacting an autoantibody in a sample with MDM2 or a MDM2 peptide fragment, and then reacting with the above anti-MDM2 antibody, followed by determining the amount of the anti-MDM2 antibody inhibited from binding MDM2, from which the amount of the autoantibody in the sample is calculated. The above anti-MDM2 antibody may be labeled with a marker before the reaction, or is subjected to the reaction followed by reacting with a marker-labeled antibody to the above anti-MDM2 antibody to determine the amount of the above anti-MDM2 antibody inhibited from binding MDM2.

The sandwich assay can be carried out if an autoantibody is a bivalent antibody that has two sites to react with an antigen. The autoantibody in a sample is sandwiched between an immobilized MDM2 or a MDM2 peptide fragment and a marker-labeled MDM2 or a MDM2 peptide fragment or a marker-labeled antibody to the autoantibody, and its amount is determined by using the marker. The amount of the autoantibody in the test sample is calculated from the amount determined above.

The sample contains a large amount of the other antibodies than the autoantibody to MDM2, which absorb or bind non-specifically to the solid phase. Therefore, if a sample from human is subjected to the measurement wherein a labeled antibody is used, the other antibody than an anti-human antibody is preferable as the labeled antibody for an accurate measurement.

The usable marker includes a radioactive substance such as $^{125}$I and $^3$H, an enzyme such as peroxidase, β-galactosidase and alkaliphosphatase, a fluorescent substance and a luminescent substance. A labeling method is suitably selected depending on a marker and a substance to be labeled. The marker may be coupled directly with the substance to be labeled, or indirectly by applying the biotin-avidin reaction for example.

An antigen used in the immunoassay of the present invention will be described below.

MDM2 used as an antigen in the present invention may be an extracted and purified product originating from a tissue, an artificial product produced by a gene recombination technique or a chemical synthesis, or a modified MDM2 within maintaining the function to bind to the autoantibody of MDM2.

A MDM2 peptide fragment used as an antigen in the present invention may be any fragment of the amino acid sequence of MDM2, regardless of the place in MDM2 amino acid sequence or the peptide length as long as it is a peptide fragment of MDM2, or further may be a modified MDM2 peptide fragment within having the function to bind to the autoantibody of MDM2. The MDM2 peptide fragment may be a product obtained by appropriate clipping from MDM2 with an enzyme or others, or a product artificially produced by applying a gene recombination technique or a chemical synthesis, as described for MDM2. The MDM2 peptide fragment has preferably about 5-30 amino acids, more preferably about 10-20 in view of antigenic property or usability.

The above modified MDM2 or modified MDM2 peptide fragment includes proteins and peptides obtained by substituting some of the amino acids in the MDM2 amino acid sequence with the other amino acids, those obtained by deleting some of the amino acids from the MDM2 amino acid sequence, and those obtained by adding some amino acids to the MDM2 amino acid sequence, unless the modifications weaken remarkably the function to bind to the autoantibody of MDM2. The phrase "weaken remarkably the function to bind to the autoantibody of MDM2" means to weaken the function so much that the object of the present invention is unattainable.

The peptide fragment is preferable in view of availability, stable quality and simplicity to establish the measurement system. The chemically synthesized peptide fragment is generally more preferable. The peptide fragment, especially the chemically synthesized peptide fragment, is easily available because it neither needs a complicated process for expressing the protein nor a process for extracting from a tissue and purifying. With respect to the stable quality, it is always available at a constant purity.

The peptide fragment can be produced, for example, by selecting about 10-20 residues in the known amino acid sequence of human MDM2, if necessary, by deleting amino acids in a part of the residue or substituting it with the other amino acids or adding one to some amino acids to the residue, and synthesising it chemically by the Fmoc method etc. One of the preferable example is a peptide fragment of 10-20 amino acid residues in the N-terminal region of human MDM2, more concretely a peptide fragment as described in the above (a) (MDM2/N, as will be defined later) of No.3 to 22 residues from the N-terminus.

The above peptide (a) of the present invention is a peptide fragment of No.3 to 22 residues from the N-terminus in the N-terminal region of human MDM2 (MDM2/N), while the above peptide (b) is a modified MDM2/N peptide fragment within the maintained function to bind to the autoantibody of MDM2. Concretely, the peptide (a) has the amino acid sequence as shown by SEQ No.1 in SEQUENCE LISTING, while the peptide (b) has an amino acid sequence derived by the deletion, substitution or addition of amino acids in a part of the amino acid sequence as shown by SEQ No.1 in SEQUENCE LISTING and maintains a function to bind to the autoantibody of MDM2. The peptide of the region (a) has a relatively high function to bind to the autoantibody of MDM2 in the amino acid sequence of human MDM2 and is also predicted to have high hydophilicity.

For the measurement of the autoantibody to MDM2 in a test sample by an immunoassay, MDM2 or a MDM2 peptide fragment as an antigen may be immobilized on a solid-phase to measure. A known method for the immobilization of a protein may be applied as the immobilization method. For example, physical adsorption or bond of MDM2 or its protein fragment by covalent bond formed with a crosslinking agent or the biotin-avidin bond to a solid-phase such as a microplate, a polystyrene bead or a magnetic fine particle, can be utilized. If necessary, the bond to a solid-phase is able to strengthen by introduction of a functional group or a linker, or it is possible to maintain the reactivity with the antibody by immobilizing in a configuration desirable for the antigen-antibody reaction.

The reagent for detecting a cancer of the present invention comprises MDM2, its peptide fragment or a labeled product thereof and an anti-MDM2 antibody if necessary. The target cancer to detect is preferably the above epithelial cancer. The MDM2 peptide fragment comprised in the reagent for detecting a cancer of the present invention includes the same peptide fragment as the MDM2 peptide fragment mentioned above. The preferable example includes a peptide fragment in the N-terminal region of MDM2, concretely a peptide which has the amino acid sequence as shown by SEQ No.1 in SEQUENCE LISTING or an amino acid sequence obtained by the deletion or substitution of amino acids of a part of the amino acid sequence, or addition of amino acids to a part of the amino acid sequence. The MDM2 peptide fragment may be immobilized to a solid-phase by the same method as described above. The present invention includes a qualitative reagent for judging existence of a cancer by a signal such as a coloration that can occur in the presence of not less or not more than a certain concentration of the autoantibody.

The kit for detecting a cancer of the present invention is a kit comprising the reagent for detecting a cancer comprising MDM2, a MDM2 peptide fragment or a labeled product thereof. The reagent for detecting a cancer comprising MDM2, a MDM2 peptide fragment or a labeled product thereof includes the above reagent. The kit has different components according to the principle for the assay such as a competition assay, an inhibition assay and a sandwich assay, and may comprise a standard solution for making a calibration curve with respect to the concentration of the autoantibody to MDM2, a test sample-diluting solution, a material for detecting an immunoreaction such as a radioisotope, an enzyme, a fluorescent substance, a colored substance and a colloidal gold. The kit for detecting the immunoreaction with an enzyme may comprise a substrate solution, a stop solution of enzymatic reaction, and a wash solution when the kit uses a solid-phase, that require B/F (Bind and Free) separation.

The kit of the present invention comprises of necessary components selected from the above components, and may further comprise a component that is comprised in a generally available test kit. Those reagents as components, if they are stable to store, can be assembled as it is, and, if unstable, may be assembled in the form such as lyophilized products or concentrated solutions accompanied by a restoring or diluting solution.

More concretely, in the case of a kit for determining the autoantibody to MDM2 in the test sample, by using for example a 96-well microplate on which MDM2 is immobilized, and making the autoantibody to compete with an anti-MDM2 antibody produced by immunizing a secondary animal species different from the animal species of the test sample against the immobilized antigen, it comprises the necessary reagents selected from the following components:

(1) a microplate on which MDM2 or a MDM2 peptide fragment is immobilized;
(2) Anti-MDM2 antibody solutions as a standard solution, having a few different concentrations prepared from an anti-MDM2 antibody which is produced by immunizing the same animal species as that of the test sample or a tertiary animal species different from a secondary animal species used for producing the anti-MDM2 antibody competing with the autoantibody;
(3) a buffer for diluting the test sample;

(4) an enzyme-labeled anti-immunoglobulin antibody which is specific to the secondary animal species for producing the anti-MDM2 antibody competing with the autoantibody to MDM2 containing in the test sample;

(5) a developing solution containing the substrate for the enzyme and dyestuff etc;

(6) a stop solution of enzymatic reaction; and

(7) a wash solution for washing the microplate at the intervals of successive reaction processes.

The kit of the present invention includes a qualitative kit for judging the presence of not less or not more than a certain concentration of the autoantibody to MDM2 in a test sample, in addition to a quantitative kit for measuring the concentration of the autoantibody in a test sample. Such an example includes a kit for judging existence of a cancer by appearance of a colored line in the detection zone in the case of the test sample containing the autoantibody in a certain concentration or more, and no cancer by no color in the case of the test sample containing in less than that concentration, as seen in a test paper according to immunochromatographic method using both MDM2 immobilized on a nitrocellulose membrane and a colloidal gold -labeled antibody to immunoglobulin containing in the test sample.

One of the methods for detecting a cancer of the present invention that uses the autoantibody to MDM2 as the index will be more concretely described according to processes.

The reactivity between a MDM peptide antigen and an antibody to the antigen can be confirmed by following processes for example:

1) A synthesized MDM2 peptide fragment antigen is immobilized on a micro-titerplate by physical adsorption.

2) The first reaction

A rabbit polyclonal anti-MDM2 antibody (IgG) produced by immunizing a rabbit with a MDM2 peptide fragment is added to each the well of the MDM2 peptide fragment antigen-immobilized plate to react.

3) The second reaction

Each the well is emptied of the solution by suction and then washed. An enzyme-labeled anti-rabbit IgG antibody solution is added to react.

4) The third reaction

Each the well is emptied of the solution by suction and then washed. A developing agent and a substrate are added to react.

5) Stop of the reaction

A reaction stop solution is added to stop the developing reaction.

6) Measurement of absorbance

The absorbance of each the well is measured to confirm adsorption of the peptide fragment antigen on the plate and the antibody to react specifically with the antigen.

A peptide fragment antigen can be selected by following processes for example:

1) Each of various MDM2 peptide fragment antigens is immobilized on respective well of a micro-titerplate by physical adsorption.

2) The first reaction

A patient serum that is anticipated to have a high autoantibody titer to MDM2 is added to each the well of the MDM2 peptide fragment antigen-immobilized plate to react.

3) The second reaction

Each the well is emptied of the solution by suction and then washed. A definite volume of solution containing of the rabbit polyclonal anti-MDM2 antibody (IgG) to the same MDM 2 peptide fragment antigen as the immobilized antigen in each well is added to react.

4) The third reaction

Each the well is emptied of the solution by suction and then washed. The enzyme-labeled anti-rabbit IgG antibody is added to react.

5) The fourth reaction

Each the well is emptied of the solution by suction and then washed. The developing agent and a substrate are added to react.

6) Stop of the reaction

The reaction stop solution is added to stop the developing reaction.

7) Measurement of absorbance

The absorbance of each the well is measured.

8) Selection of a peptide fragment antigen

The reactivity of each of the MDM2 peptide fragment antigens with an autoantibody in a test serum is reflected on the absorbance. The higher reactivity of a peptide fragment antigen increases the biding amount of the MDM2 autoantibody in the test serum, and, as the result, decreases the amount of the peptide fragment antigen to react with the above anti-MDM2 antibody, and finally decreases the amount of the anti-MDM2 antibody to couple with the immobilizedMDM2 antigen. Consequently, the binding amount of the enzyme-labeled rabbit IgG antibody to the above coupled anti-MDM2 antibody (rabbit polyclonal antibody) is decreased, resulting in a weakened coloring and a lowered absorbance. Therefore, the lower absorption is considered to indicate the higher reactivity of the antigen. An antigen giving the lowest absorbance is selected among various MDM2 peptide fragment antigens.

According to an inhibition assay, an anti-MDM2 autoantibody can be measured, for example, by following processes:

1) The MDM2 peptide fragment antigen is immobilized on the wells of a microtiter-plate by physical adsorption.
2) The first reaction
A test serum or a only buffer is added to each the well of the MDM2 peptide fragment antigen-immobilized plate to react.
3) The second reaction
Each the well is emptied of the solution by suction and then washed. The solution of the rabbit polyclonal anti-MDM2 antibody (IgG) to the MDM2 peptide fragment antigen is added to react to the well where the test serum has been reacted, while the rabbit polyclonal antibody standard solutions are added to react to the wells where the buffer has been reacted. The antibody standard solutions are prepared in a series of various concentrations of the rabbit polyclonal anti-MDM2 antibody (IgG) to the MDM2 peptide fragment, which is the same antibody as the antibody added to the well reacted with the test serum
4) The third reaction
Each the well is emptied of the solution by suction and then washed. The enzyme-labeled anti-rabbit IgG antibody solution is added to react.
5) The fourth reaction
Each the well is emptied of the solution by suction and then washed. The developing agent and a substrate are added to react.
6) Stop of the reaction
The stop solution of the reaction is added to stop the developing reaction.
7) Measurement of absorbance
The absorbance of each the well is measured.
8) The method of determination of the concentration of the autoantibody to MDM2 (hereinafter simply referred to as the MDM2 autoantibody) in blood

A calibration curve is drawn from the absorbance values of the wells to which the rabbit polyclonal antibody standard solutions are added. An absorbance of the well to which the test serum is added is applied to the calibration curve to determine the concentration of the reacted rabbit polyclonal antibody, which is inhibited to bind by the bound autoantibody. The difference between the concentration of the added rabbit polyclonal anti-MDM2 antibody and the determined concentration is ascribed to the concentration of the autoantibody to MDM2 in blood (the blood MDM2 autoantibody concentration).
The present invention will be described below in details with respect to examples, but is not limited to the examples.

EXAMPLE

Example 1. Production of MDM2 peptide fragments and selection of an antigen having a high specificity to cancer

1. Synthesis of various MDM2 peptide fragments
From the N-terminal region, the C-terminal region and the intermediate region of human MDM2 amino acid sequence, a highly hydrophilic region and a region predicted to be an antigenic determinant were selected. Three kinds of MDM2 peptide fragments having 14-20 residues were chemically synthesized by the Fmoc method. The N-terminal region-derived product is referred to as MDM2/N, the C-terminal region-derived product is referred to as MDM2/C, and the intermediate region-derived product is referred to as MDM2/NC.
2. Preparation of a MDM2 antigen-immobilized microtiter-plate
Said three kinds of MDM2 peptide fragments dissolved in 0.1M carbonate buffer (pH9.0) were added to the each well of a micro titer-plate (trade name: AquaBind, Denmark M&E Co.) in amount of 5 μg/150 μl/well. After

reacting at room temperature for 3 hours, each the well was emptied of the antigen solution by suction and washed twice with 1 ml of 0.1M carbonate buffer (pH9.0) by a microplate washer (Life Tec Co.,trade name: Minilabo Washer). Then, 0.05M phosphate buffered physiological saline (pH7.4, PBS) containing 15% polyethylene glycol(MW 4,000), 10mM diethanol amine and 0.1% sodium azide was added at a volume of 250 μl/well. The wells were tightly sealed, left to stand at room temperature for 4 hours, and stored at 4°C until the measurement was carried out.

3. The first reaction

Said each the well of the MDM2 antigen-immobilized microtiter-plate was emptied of the solution by suction. A patient serum that was anticipated to have a high MDM2 autoantibody titer was diluted 9-fold with 0.05M PBS (pH7.4) containing 0.02% Antifoam A (Trade name). To each the well was added the resultant solution in amount of 100μl/well to react at room temperature for 2 hours.

4. The second reaction

After the first reaction, each the well was emptied of the solution by suction, and washed twice with 2 ml of physiological saline containing 0.05% Tween20 (Trade name) by the microplate washer. The rabbit polyclonal anti-MDM2 antibodies (IgG)(See Example 2-1 as described later) to their respective immobilized peptide fragment antigens, whichwere produced by using their respective MDM2 peptide fragments as their respective immunogens, were then diluted at concentration of 20 ng/ml with 0.05M PBS (pH7.4)containing 1% normal rabbit serum. To each the well was added the solution in amount of 100 μl/well to react at room temperature for 2 hours.

5. The third reaction

After the second reaction, each the well was emptied of the solution by suction, and washed twice with 2 ml of physiological saline containing 0.05% Tween20 (Trade name) by the microplate washer. Peroxidase conjugated monoclonal rat anti-rabbit IgG (ZYMED Lab., INC) was then diluted 1,000 -fold (450 ng/ml) with 0.05M PBS (pH7.4) containing 1% rat serum. To each the well was added the resultant solution in amount of 100μl/well to react at room temperature for 45 minutes.

6. The fourth reaction

After the third reaction, each the well was emptied of the solution by suction, and washed twice with 2 ml of physiological saline containing 0.05% Tween20 (Trade name) by the microplate washer. The developing agent, ABTS (2,2'-azinobis {3-ethylbenzothiazoline-6-sulfonic acid} diammonium salt, Nakaraitesuku KK) was then dissolved at concentration of 3 mg/ml with 0.1M citrate phosphate buffer (pH4.0). Aqueous hydrogen peroxide solution (20%) as a substrate was added to the solution at concentration of 0.04%. To each the well was added the resultant solution in amount of 100μl/well to react at room temperature for 30 minutes.

7. Stop of the reaction

After the fourth reaction, 0.1M citrate phosphate buffer (pH4.0) containing 0.1% sodium azide was added to each the well in amount of 100μl/well to stop the reaction. The absorbance of each the colored solution in the well was measured at a wavelength of 420nm.

8. Selection of MDM2 peptide fragment antigen

The MDM2/N, which showed the lowest absorbance, was selected from the MDM2 peptide fragment antigens to use for the measurement of MDM2 autoantibody in a test sample, as shown later in Example 2.

Example 2 Measurement of MDM2 autoantibody by inhibition assay

A rabbit polyclonal anti-MDM2 antibody to the MDM2/N antigen prepared in Example 1 was produced, and then MDM2 autoantibody was measured by the inhibition assay.

1. Production of a rabbit polyclonal anti-MDM2 antibody (IgG)

The MDM2/N antigen prepared in Example 1 was mixed with an equivalent amount of Freund's Complete Adjuvant and emulsified thoroughly, followed by administering to a rabbit to immunize. The immunization was carried out every two weeks. After four months, an antiserum obtained by collecting of blood was fractionated with 40% ammonium sulfate, then dialyzed and condensed. A rabbit polyclonal anti-MDM2 antibody (IgG) was obtained by a protein A affinity chromatography.

2. Preparation of a MDM2/N antigen-immobilized microtiter-plate

The MDM2/N produced in Example 1 dissolved in 0.1M carbonate buffer (pH9.0) added to the wells of a micro titer-plate (trade name: AquaBind, Denmark M&E Co.)in amount of 5 μg/150 μl/well. After reacting at room temperature for 3 hours, each the well was emptied of the antigen solution by suction and washed twice with 1 ml of 0.1M carbonate buffer (pH9.0) by a microplate washer (Life Tec Co.,trade name: Minilabo Washer). Then, 250 ml of 0.05M PBS (pH7.4) containing 15% polyethylene glycol(MW 4,000), 10mM diethanol amine and 0.1% sodium azide was added in each well. The wells were tightly sealed, left to stand at room temperature for 24 hours, and stored at 4°C until the measurement was carried out.

3. Preparation of anti-MDM2 antibody standard solution

From the rabbit polyclonal anti-MDM2 antibody (IgG) produced above using MDM2/N as the antigen, anti-MDM2 antibody standard solutions were prepared by the following way. Namely the rabbit polyclonal anti-MDM2 antibody (IgG) was diluted with 0.05MPBS (pH7.4) containing 1% normal rabbit serum to 20 ng/ml, and the resultant solution was further diluted twice by twice to 1.25 ng/ml with the same buffer to get the five concentrations of anti-MDM2 antibody standard solutions. Fifty millimole per liter of 0.05M PBS (pH7.4) containing 1% normal rabbit serum was directly used as 0 ng/ml of anti-MDM2 antibody standard solution without further preparation.

4. Measurement of MDM2 autoantibody in blood

1) The first reaction

Each the well of the MDM2/N antigen-immobilized micro titer-plate as prepared in the above section 2 was emptied of 0.05M PBS (pH7.4) containing 15% polyethylene glycol(MW 4,000), 10mM diethanol amine and 0.1% sodium azide by suction. To each the well (well for the anti-MDM2 antibody standard solution), in which the anti-MDM2 antibody standard solution instead of the test serum would be added in the succeeding second reaction, was added 0.05M PBS (pH7.4) containing 0.1% sodium azide in amount of 100μl/well. To each the well other than said well was added the test serum diluted 9-fold with 0.05M PBS (pH7.4) containing 0.02% Antifoam A (trade name) in amount of 100 μl/well followed by reacting at room temperature for 2 hours.

2) The second reaction

After the first reaction, each the well was emptied of the solution by suction, and washed twice with 2 ml of physiological saline containing 0.05% Tween20 (Trade name). Hundred micro liter of 20ng/ml anti-MDM2 antibody standard solution prepared in the above 3 was added to each the well in which the test serum was reacted in the first reaction. Each concentration of anti-MDM2 antibody standard solutions prepared in the above 3 was added to respective wells for the anti-MDM2 antibody standard solution described in the first reaction paragraph in amount of 100 μl/well. Then they react at room temperature for 2 hours.

3) The third reaction

After the second reaction, each the well was emptied of the solution by suction, and washed twice with 2 ml of physiological saline containing 0.05% Tween20 (Trade name) by the microplate washer. And then, Peroxidase conjugated monoclonal rat anti-rabbit IgG (ZYMED Lab., INC) that was diluted 1,000 -fold (450 ng/ml) with 0.05M PBS (pH7.4) containing 1% rat serum was added to each the well in amount of 100 μl/well to react at room temperature for 45 minutes.

4) The fourth reaction

After the third reaction, each the well was emptied of the solution by suction, and washed twice with 2 ml of physiological saline containing 0.05% Tween20 (Trade name) by the microplate washer. The developing agent, ABTS (2,2'-azinobis {3-ethylbenzothiazoline-6-sulfonic acid} diammonium salt, Nakaraitesuku KK) was then dissolved in 0.1M citrate phosphate buffer (pH4.0) at concentration of 3 mg/ml and 20% aqueous hydrogen peroxide solution as a substrate was added to the solution at concentration of 0.04%. The resultant solution was added to each the well in amount of 100 μl/well to react at room temperature for 30 minutes.

5) Stop of the reaction

After the fourth reaction, 0.1M citrate phosphate buffer (pH4.0) containing 0.1% sodium azide was added to each the well in amount of 100 μl/well to stop the reaction.

6) Measurement of absorbance

The absorbance of each the colored solution in well was measured at a wavelength of 420nm.

7) Determination of the concentration of MDM2 autoantibody

The absorbances of the anti-MDM2 antibody standard solutions were used to draw a calibration curve, to which the absorbance of each the well was applied to determine the concentration inhibited from binding of the anti-MDM2 antibody in the 20 ng/ml anti-MDM2 antibody standard solution because of binding of the autoantibody in the test serum and the concentration is ascribed to a concentration of the MDM2 autoantibody in blood.

8) Measurement results on normal subjects and patients with various cancers from the above inhibition assay

From the measurement results of MDM2 autoantibody in blood in normal subjects, the average concentration $\pm$ 2SD(2 times of standard deviation) in normal male 20 cases were 8.2 $\pm$ 11.9 ng/ml and the measurement results were less 20 ng/ml in all the cases. On the other hand, the average concentration $\pm$ 2SD in normal female cases were 27.5 $\pm$ 18.6 ng/ml and the measurement results were 15 ng/ml and more in 19 of 20 cases, which was significantly higher than that in male. In consideration of the significant difference in the distribution of the determined antibody values between normal males and females, a provisional cut-off value was decided to be 15 ng/ml in both male and female. A male, if he had the cut-off value or more, was classified to be positive to calculate a positive rate. A female, if she had the cut-off value or less, was classified to be positive to calculate a positive rate. The determined anti-MDM2 autoantibody value in each test sera was summed up separately on male and female.

Table 1

| Measurement results of MDM2 autoantibodies in male patients with various cancers | | | | |
|---|---|---|---|---|
| Disease | Number of cases | Average ± 2SD ng/ml | Positive rate % | Significant test (to Normal male) |
| Normal | 20 | 8.2±11.9 | 10 | - |
| Large intestinal cancer | 11 | 24.9±17.4 | 100 | P<0.001 |
| Gastric cancer | 19 | 20.6±22.8 | 68 | P<0.001 |
| Pancreatic cancer | 4 | 15.7±7.9 | 50 | P<0.01 |
| Hepatocellular carcinoma | 4 | 29.0±23.8 | 75 | P<0.05 |
| Lung cancer | 11 | 28.5±22.0 | 82 | P<0.001 |

Table 2

| Measurement results of MDM2 autoantibodies in female patients with various cancers | | | | |
|---|---|---|---|---|
| Disease | Number of cases | Average ± 2SD ng/ml | Positive rate % | Significance test (to Normal female) |
| Normal | 20 | 27.5±18.6 | 10 | - |
| Breast cancer | 27 | 13.2±22.5 | 52 | P<0.001 |
| Large intestinal cancer | 5 | 22.5±20.0 | 40 | NS |
| Gastric cancer | 3 | 21.2±35.5 | 33 | NS |
| Pancreatic cancer | 1 | 20.3 | 0 | NS |
| Hepatocellular carcinoma | 2 | 18.8±5.6 | 0 | NS |
| Ovarian cancer | 2 | 19.7±1.8 | 0 | NS |
| Lung cancer | 4 | 26.9±26.0 | 25 | NS |
| NS: no significant difference | | | | |

The average ± 2SD in the determined values of MDM2 autoantibodies in blood were shown in male and female respectively in Table 1 and Table 2. The data shows a trend that the value of average ± 2SD in the male is statistically significantly higher in large intestinal cancer, gastric cancer, pancreatic cancer, hepatocellular carcinoma and lung cancer than in normal cases. On the other hand, in this Example the female had a significantly higher value of average ± 2SD in no cancer than in normal cases. On the contrast, the data shows a trend that the value of average ± 2SD of the patients with breast cancer is rather statistically significantly lower than the normal females (P<0.001).

Positive rates in male blood MDM2 autoantibody are 100% in 11 cases of large intestinal cancer, 68% in 19 cases of gastric cancer, 50% in 4 cases of pancreatic cancer, 75% in 4 cases of hepatocellular carcinoma and 82% in 11 cases of lung cancer. Positive rates of the male patients with cancers are sufficiently high compared with that of the normal males of which the positive rate was 10%.

Example 3 Measurement of MDM2 autoantibody by a competition assay (1)

MDM2 autoantibody was measured by a competition assay using a biotinylated MDM2/N having biotinylated N-terminus.

1. Preparation of an avidin-immobilized microplate

Avidin (Nakaraitesuku KK) was dissolved in 30 μg/ml in 0.1M sodium bicarbonate buffer (pH9.6). The resultant solution was added in each the well of a microplate (1×8 stripwell plate, CORNING Costar Co.) in amount of 100 μl/well and left to stand at 4°C overnight, and then the avidin solution was removed. 0.05M PBS (pH7.2) containing 1% bovine serum albumin was added in each the well at a volume of 300 μl/well, which was sealed, left to stand at 4°C overnight and then stored at 4°C until the measurement was carried out.

2. Preparation of the biotinylated MDM2/N having biotinylated N-terminus

The N-terminal amino acid (asparagine) of the above MDM2/N was biotinylated with N-succinimidyl D-biotin according to a conventional method to produce the biotinylated MDM2/N peptide. The peptide was purified by a reversed phase HPLC method to get the biotinylated MDM2/N having a purity of 97%.

3. Measurement of MDM2 autoantibody in blood

1) The first reaction

A test serum was diluted 9-fold with 0.05M PBS (pH7.4) containing 2% dextran 70,000 (Tokyo Kasei), 3% bovine serum albumin and 0.9% Triton X-100 (trade name) to get a test sample-diluted solution. The solution was put into a test tube in amount of 100ml. The rabbit polyclonal anti-MDM2 antibody (IgG) produced in Example 2 was diluted with 0.05M PBS (pH7.4) containing 2% dextran 70,000 (Tokyo Kasei), 3% bovine serum albumin and 0.9% Triton X-100 (trade name) to 20 ng/ml. The obtained competitive antibody solution (100 µl) was added to the above test sample-diluted solution in the test tube. Furthermore, 100 µl of 0.05M PBS (pH7.4) containing the biotinylated MDM2/N having biotinylated N-terminus at 19.5 ng/ml was added and mixed immediately, followed by leaving to stand at room temperature for 2 hours.

2) The second reaction

The above avidin-immobilized microplate was left up to room temperature. The well was emptied of the solution by suction and washed thrice with 3 ml of a phosphate buffered physiological saline (pH7.4) containing 0.05% Tween 20 (trade name). The reaction solution after completion of the first reaction was added in amount of 100 µl /well in each the well, which was set in a microplate incubator (TOMY Co.) to react at 30°C for 90 minutes.

3) The third reaction

After completion of the second reaction, the well was emptied of the solution by suction, and washed thrice with 3 ml of PBS (pH7.4) containing 0.05% Tween 20 (Trade name) by the microplate washer. Peroxidase conjugated monoclonal rat anti-rabbit IgG (ZYMED Lab., INC) was then diluted 500-fold (0.9 ng/ml) with 0.05M PBS (pH7.4) containing 1% rat serum and 1% bovine serum albumin. The resultant solution was added in amount of 100 µl/well to each the well, which was set in the microplate incubator to react at 30°C for 45 minutes.

4) The fourth reaction

After completion of the third reaction, the well was emptied of the solution by suction, and washed thrice with 3 ml of PBS (pH7.4) containing 0.05% Tween 20 (Trade name) by the microplate washer. The developing agent ABTS was then dissolved in concentration of 3 mg/ml in 0.1M citrate phosphate buffer (pH4.0). 20% aqueous hydrogen peroxide solution as a substrate was added to the solution for its concentration to be 0.04%. The resultant solution was added in amount of 100 µl /well to each the well to react at room temperature for 10 minutes.

5) Stop of the reaction

After completion of the fourth reaction, an aqueous sodium azide solution was added in amount of 100 µl /well to each the well to stop the reaction.

6) Measurement of absorbance

The absorbance of each the well was measured at a wavelength of 415nm.

7) Determination of the concentration of MDM2 autoantibody in a test sample

According to this assay, the higher concentration of MDM2 autoantibody in a test sample decreases the absorption. Therefore, using the absorbance obtained by measurment of a MDM2 autoantibody free sample as standard, the inhibition rate of the color development was defined as a measurement value of the autoantibody concentration. The measurement value as defined by the following equation was deemed to show a autoantibody concentration:

$$\text{Measurement value (\%)} = \{(A\text{-}C)/(A\text{-}B)\} \times 100$$

wherein A is an absorbance obtained by using the test sample-diluted solution free from a test serum and an a competitive antibody solution comprising 20 ng/ml rabbit polyclonal anti-MDM2 antibody (IgG); B is an absorbance obtained by using the test sample-diluted solution free from a test serum and a competitive antibody solution free from rabbit polyclonal anti-MDM2 antibody; and C is an absorbance obtained by using the test sample-diluted solution comprising a test serum and an a competitive antibody solution comprising 20 ng/ml rabbit polyclonal anti-MDM2 antibody (IgG).

8) Measurement result

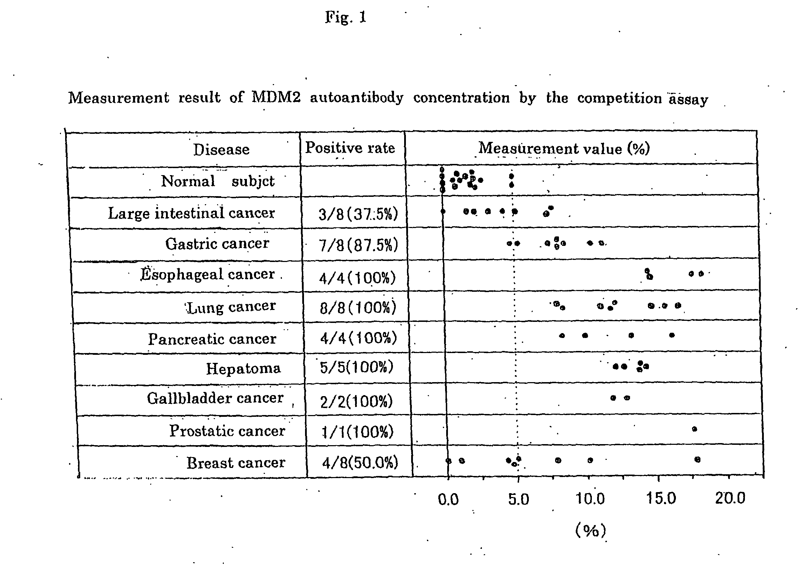

The result of measurement by the competition assay is shown in Fig. 1. In this example, the normal subjects were shown no significant difference in measurement values between both groups of 8 males and 8 females. The inhibition rate was 5% and less in the MDM2 autoantibody concentration in blood of the normal subjects. Hence, a cut-off value was set to be 5.1% and more. The patients with cancers had the high inhibition rates regardless of their sexes. The positive rates in cancers were 37.5% in large intestinal cancer, 87.5% in gastric cancer, 100% in esophageal cancer, 100% in lung cancer, 100% in pancreatic cancer, 100% in hepatoma, 100% in gallbladder cancer, 100% in prostatic cancer and 50% in breast cancer. The data showed good performance.

Example 4 Measurement of MDM2 autoantibody by a competition assay (2)

The measurement by a competition assay in a liquid phase was studied in Example 3. This example is a measurement method based on a competition reaction between a biotinylated rabbit polyclonal anti-MDM2 antibody and a MDM2 autoantibody existing in test serum to the immobilized MDM2/N antigen. This method was studied by using the rabbit polyclonal anti-MDM2 antibody as the standard antibody and a test serum different from the test serum used in Example 3.

1) Preparation of a biotinylated rabbit polyclonal anti-MDM2 antibody

The rabbit polyclonal anti-MDM2 antibody produced in Example 2 was dissolved in 0.05M PBS buffer solution (pH7.8) at a concentration of 50 µg/250 µl. A reagent for introducing a biotinyl group into an amino group, EZ-Link(TM) Sulfo-NHS-LC-Biotin (PIERCE Co.) was dissolved in distilled water at a concentration of 2 mg/50µl. Resultant both solutions were reacted in a glass tube at a room temperature of 25°C for 3 hours under shielding from light. After the reaction, the reacted solution was passed through the D-Salt™ Desalting Column (PIERCE Co.) to terminate the reaction. The excess EZ-Link™ Sulfo-NHS- LC-Biotin reagent, the non-biotinylated rabbit polyclonal anti-MDM2 antibody and the biotinylated rabbit polyclonal anti-MDM2 antibody were eluted with 0.05M PBS buffer solution(pH7.4) to separate (0.5 ml/fraction). The biotinylated rabbit polyclonal anti-MDM2 antibody fractions were selected from the column chromatography fractions by a following way.

Each of the column chromatography fractions was diluted 500-fold with a buffer solution containaing 1% fetal calf serum (FCS), which is the same buffer solution as used in the elution of the each fraction. The diluted solution was added in amount of 100 µl/well in each the well of the MDM2/N antigen-immobilized micro titer-plate prepared in Example 2 to react at 30°C for 60 minutes. After completion of the reaction, each the well was emptied of the reaction solution by suction and washed thrice with a physiological saline containing 0.02% Tween 20 (Trade name). The alkaliphosphatase-labeled streptoavidin (ZYMED Lab., INC.) diluted to a concentration of 3.0 µg/ml with 50mM Tris-HCl buffer solution (pH8.0) containing 1% FCS, 1mM $MgCl_2$, 0.9% NaCl and 0.1% sodium azide was added in an amount of 100 µl/well in the each well to react at 30°C for 30 minutes. After completion of the reaction, each the well was emptied of the reaction solution by suction and washed thrice with a physiological saline containing 0.02% Tween 20 (Trade name). Disodium p-nitrophenyl phosphate (Nakarai tesuku KK) diluted to a concentration of 4.0 mg/ml with 0.1M carbonate buffer solution (pH9.6) containing 1mM $MgCl_2$ was added in amount of 100 µl/well in each the well to react at 30°C for 30 minutes. After completion of the reaction, 1N sodium hydroxide was added in amount of 100 µl /well to each the well to stop the reaction. The absorbance of each the well was measured at a wavelength of 405nm. Fractions having high absorbance were selected and used for assaying as a biotinylated rabbit polyclonal anti-MDM2 antibody.

2) The first reaction

Each the test serum or each the rabbit polyclonal anti-MDM2 antibody having seven different concentrations in a series from 32 µg/ml to 0.5 µg/ml for preparing a calibration curve were diluted 9-fold with the biotinylated rabbit polyclonal anti-MDM2 antibody solution having a concentration of 133 ng/ml by diluting with 0.05M PBS buffer solution (pH7.4) containing 0.1% normal rabbit serum. Each 100 μl of them was added in each the well of the MDM2/N antigen-immobilized micro titer-plate prepared in Example 2, followed by reacting at 30°C for 2 hours.

3) The second reaction

After completion of the first reaction, each the well was emptied of the reaction solution by suction and washed thrice with a physiological saline containing 0.02% Tween 20 (Trade name). The alkaliphosphatase-labeled streptoavidin (ZYMED Lab., INC.) solution having a concentration of 3.0 μg/ml by diluting with 50mM Tris-HCl buffer solution (pH8.0) containing 1% FCS, 1mM $MgCl_2$, 0.9% NaCl and 0.1% sodium azide was added in amount of 100 μl in each the well to react at 30°C for 30 minutes.

4) The third reaction

After completion of the second reaction, each the well was emptied of the reaction solution by suction and washed thrice with a physiological saline containing 0.02% Tween. 20 (Trade name). Disodium p-nitrophenyl phosphate (Nakarai tesuku KK) was diluted to a concentration of 4.0 g/ml with 0.1M carbonate buffer solution (pH9.6) containing 1mM $MgCl_2$. The resultant solution was added in amount of 100 μl/well in each the well to react at 30°C for 30 minutes.

5) Stop of the reaction

After completion of the third reaction, 1N sodium hydroxide was added in amount of 100 μl/well to each the well to stop the reaction.

6) Measurement of absorbance

The absorbance of each the well was measured at a wavelength of 405nm.

7) Determination of the concentration of MDM2 autoantibody

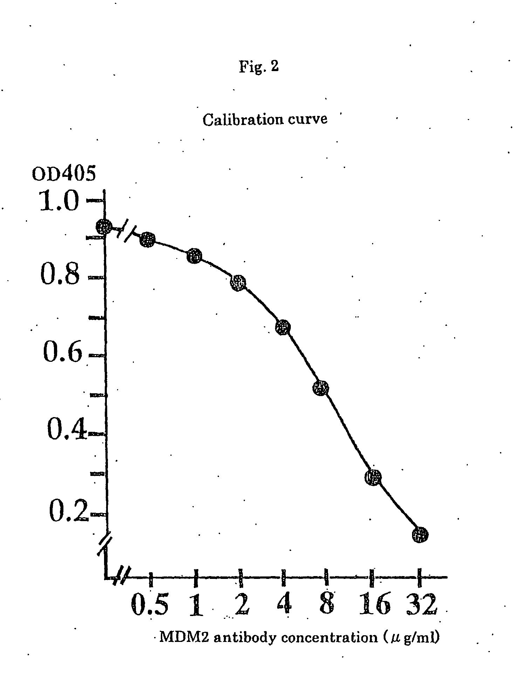

A calibration curve was drew by taking the measured absorbances of the standard rabbit polyclonal anti-MDM2 antibody solutions of seven different concentrations in a series for preparing a calibration curve to the vertical axis and their concentrations to the horizontal axis.
One of the examples is shown in Fig. 2. The measured absorbances of the test sera were applied to the calibration curve to determine the concentrations of MDM2 autoantibodies in the test sera.

8) Measurement result

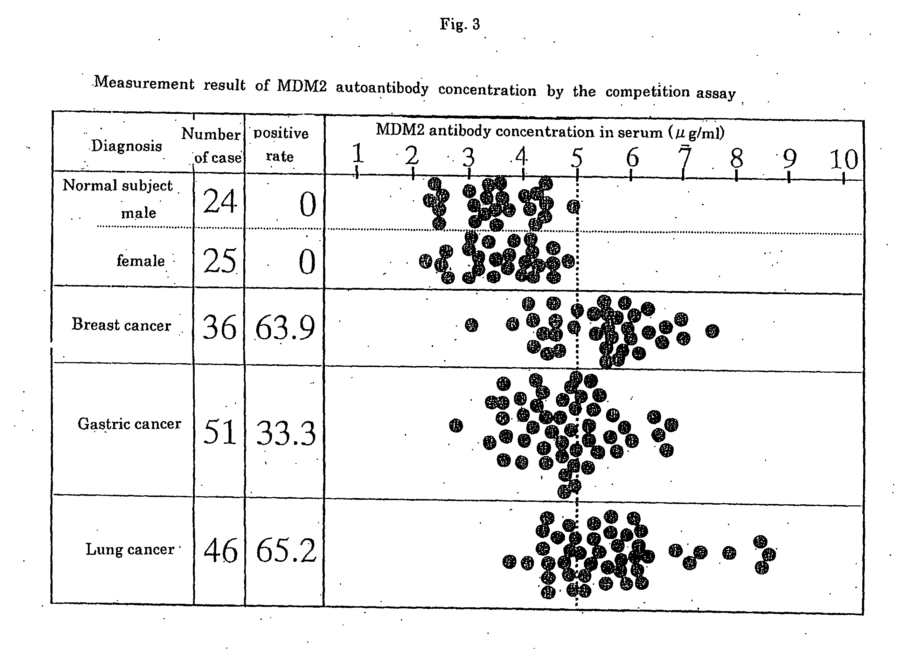

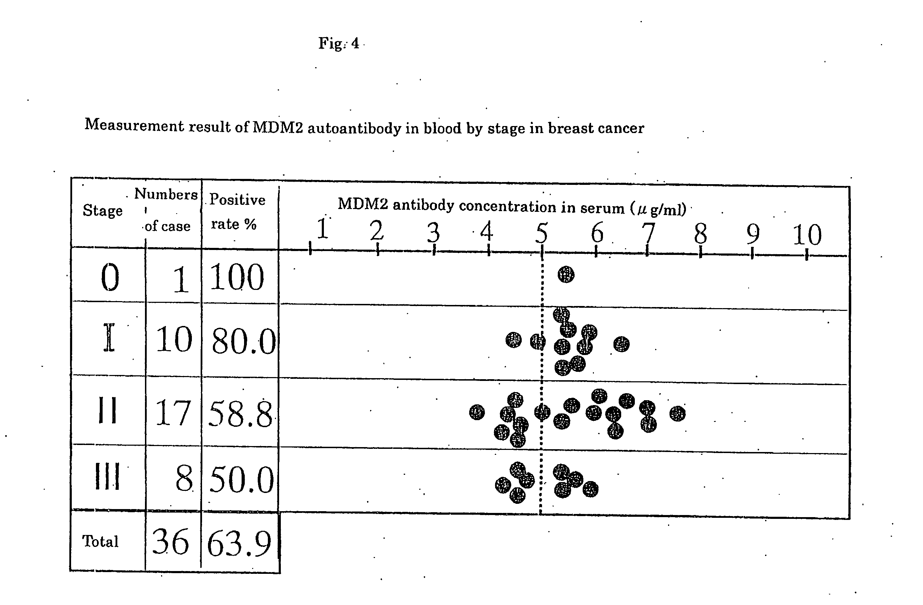

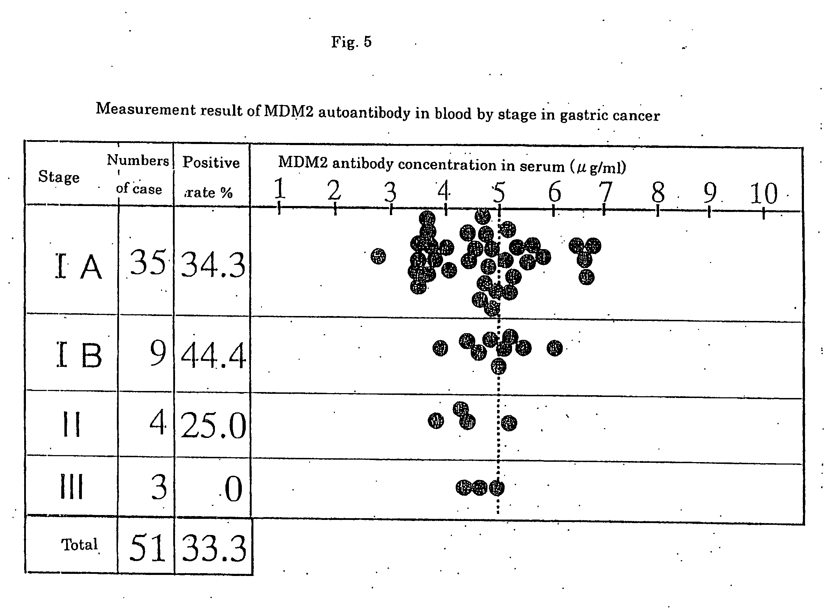

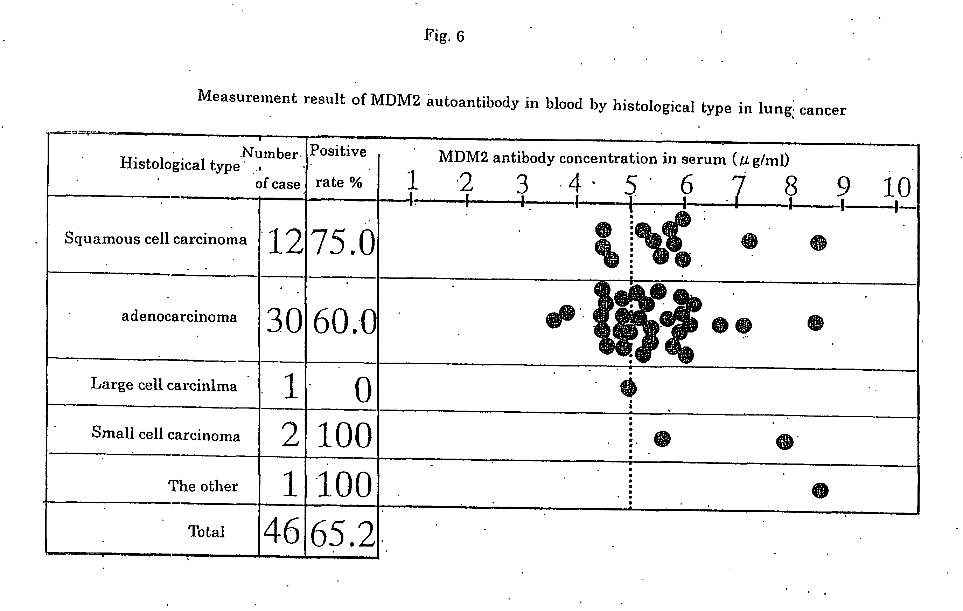

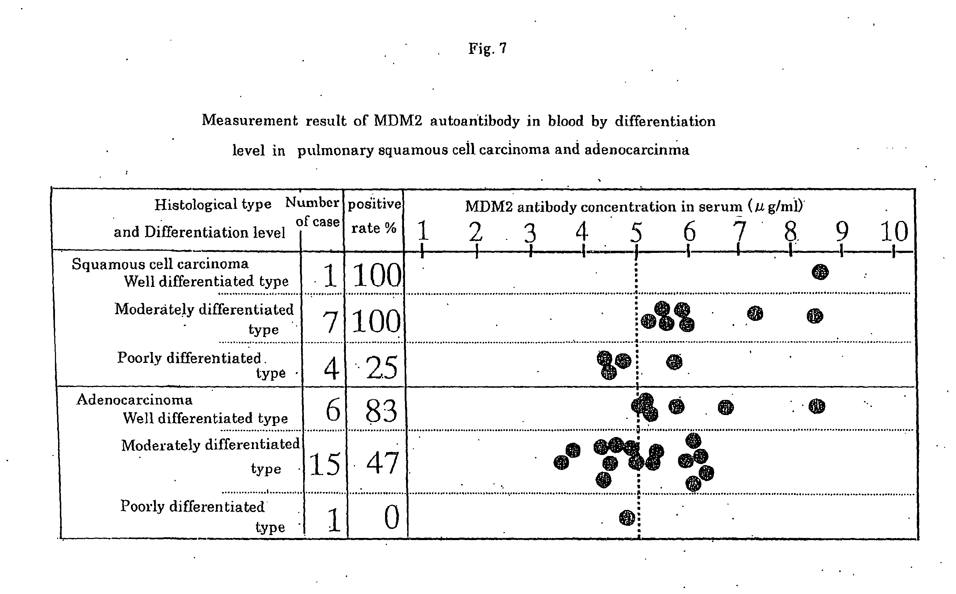

The measurement result of MDM2 autoantibody in blood is shown in Fig.3. The MDM2 autoantibody in blood in total 49 cases of 24 normal males and 25 normal females was 5 μg/ml and less. When this value was decided as a cut-off value, the positive rates for MDM2 autoantibody in blood in the patients with cancers were 63.9% in breast cancer (23/36), 33.3% in gastric cancer (17/51) and 65.2% in lung cancer (30/46).
The positive rates for MDM2 autoantibody in blood by stage in breast cancer and gastric cancer are shown in Fig.4 and Fig.5. The positive rates in breast cancer were 100% in Stage 0 (1/1), 80% in Stage I (8/10), 58% in Stage II (10/17), and 50% in Stage III (4/8). Those in gastric cancer were 34.3% in Stage IA (12/35), 44.4% in Stage IB(4/9), 25% in Stage II(1/4), and 0% in Stage III(0/3). It was revealed that the Stages 0 and I in breast cancer as well as the Stages I A and IB in gastric cancer showed a trend of the higher positive rates than their Stages IIs and IIIs. It is suggested that the measurement of MDM2 autoantibody is useful for early diagnosis of breast cancer and gastric cancer.bound
The measurement result of MDM2 autoantibody in blood by histological type in lung cancer is shown in Fig. 6. The positive rates were 75% in squamous cell carcinoma (9/12), 60% in adenocarcinoma (18/30), 100% in small cell carcinoma (2/2), and 0% in large cell carcinoma (0/1). The measurement result of MDM2 autoantibody in blood by differentiation level in pulmonary squamous cell carcinoma and pulmonary adenocarcinoma is shown in Fig.7. The positive rates of MDM2 autoantibody in blood by differentiation level in pulmonary squamous cell carcinoma were 100% in well differentiated type(1/1), 100% in moderately differentiated type(7/7), and 25% in poorly differentiated type (1/4). Those in pulmonary adenocarcinoma were 83% in well differentiated type (5/6), 47% in moderately differentiated type (7/15), and 0% in poorly differentiated type (0/1). The well differentiated types in pulmonary squamous cell carcinoma and pulmonary adenocarcinoma showed a trend of the higher positive rates than their poorly differentiated types. It was further revealed from the result of immunohistologic staining using the rabbit polyclonal anti-MDM2 antibody in said cases of pulmonary squamous cell carcinoma and pulmonary adenocarcinoma that expressions of the MDM2 antigen were the higher in the well differentiated types than in the poorly differentiated types.

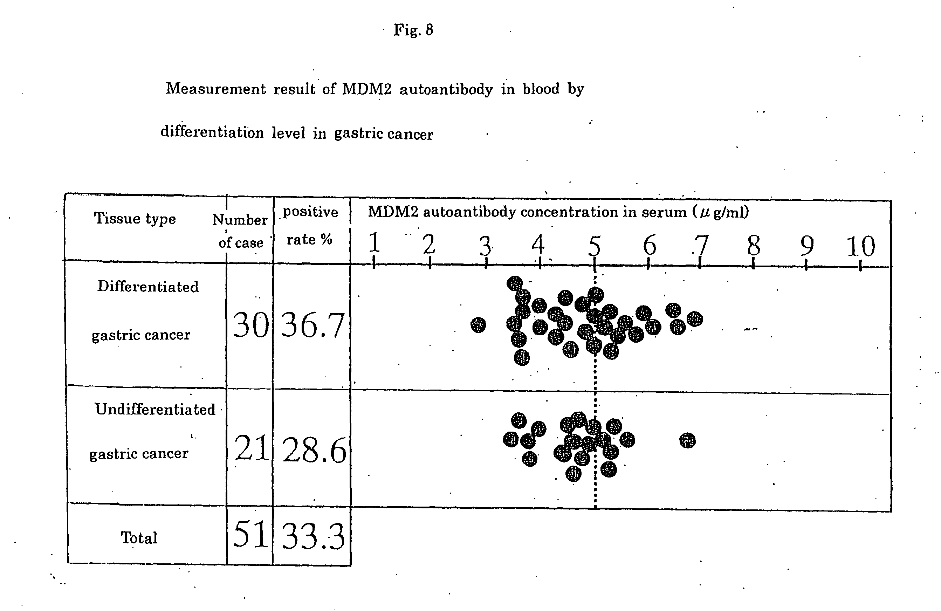

The measurement result of MDM2 autoantibody in blood by differentiation level in the gastric cancer is shown in Fig.8. The positive rates of MDM2 autoantibody in blood by differentiation level in gastric cancer were 36.7% in differentiation type (11/30), and 28.6% in undifferentiation type (6/21). The differentiation types show a trend of a slightly higher positive rate than the undifferentiated type in gastric cancer.

This example as well as Example 3 reveals that the patients with cancers show higher positive rates than the normal subjects, also, and that the present invention enables diagnosis of cancers.

INDUSTRIAL APPLICABILITY

The present invention enables a simple and high sensitive quantitative measurement of an autoantibody to MDM2 in blood, the value of which is utilized as an index for a specific and early diagnosis of a cancer.

The autoantibody to MDM2, though it can be found in the blood of a normal human, increases remarkably in the blood of a patient with a cancer in general, and hence can be used for the diagnosis of a cancer. The distribution of the concentration of an autoantibody to MDM2 in blood is different that in patients with cancers from that in the normal humans. Therefore, if a certain value (cut-off value) is determined previously, it is able to detect a cancer by a higher value than the prescribed value in diagnosis.

**Claims**

1. A method for detecting a cancer **characterized by** measuring autoantibody to MDM2 existing in a test sample.

2. The method according to Claim 1, wherein said cancer is an early stage of cancer.

3. The method according to Claim 1, wherein said cancer is an epithelial cancer.

4. The method according to Claim 3, wherein said epithelial cancer is pulmonary squamous cell carcinoma, pulmonary adenocarcinoma or pulmonary small cell carcinoma.

5. The method according to any one of Claim 1 to 4, wherein said measurement of the autoantibodies is carried out by an immunoassay.

6. The method according to Claim 5, wherein said immunoassay is a competition assay, an inhibition assay or a sandwich assay.

7. The method according to Claim 5, wherein said immunoassay utilizes a peptide fragment of MDM2 (hereinafter referred to as a MDM2 peptide fragment) as an antigen for the immunoassay.

8. The method according to Claim 5, wherein said immunoassay uses an anti-MDM2 antibody or the labeled product thereof.

9. The method according to Claim 7, wherein said MDM2 peptide fragment is an N-terminal region peptide fragment of MDM2 or the modified product thereof.

10. The method according to Claim 7 or 9, wherein said MDM2 peptide fragment is immobilized on a solid-phase.

11. A peptide as defined by the following (a) or (b):

    (a) a peptide which has the amino acid sequence as shown by SEQ No.1 in SEQUENCE LISTING and has a function to bind an autoantibody to MDM2; or
    (b) a peptide which has an amino acid sequence obtained by the deletion, substitution or addition of amino acid(s) in a part of the amino acid sequence as shown by SEQ No.1 in SEQUENCE LISTING and has a function to bind an autoantibody to MDM2.

12. A reagent for detecting a cancer comprising MDM2, a MDM2 peptide fragment or their labeled product(s).

13. A reagent for detecting a cancer comprising an anti-MDM2 antibody or the labeled product thereof.

14. The reagent for detecting a cancer according to Claim 12, wherein said cancer is an epithelial cancer.

15. The reagent for detecting a cancer according to Claim 12, wherein said MDM2 peptide fragment is a N-terminal region peptide fragment of MDM2 or the modified product thereof.

16. The reagent for detecting a cancer according to Claim 12 or 15, wherein said MDM2 peptide fragment is immobilized on a solid-phase.

17. The reagent for detecting a cancer according to Claim 12, wherein said MDM2 peptide fragment is a peptide as defined by the following (a) or (b):

    (a) a peptide which has the amino acid sequence as shown by SEQ No.1 in SEQUENCE LISTING and has a function to bind an autoantibody to MDM2; or
    (b) a peptide which has an amino acid sequence obtained by the deletion, substitution or addition of a part of amino acid(s) in the amino acid sequence as shown by SEQ No.1 in SEQUENCE LISTING and has a function to bind an autoantibody to MDM2.

18. A kit for detecting a cancer comprising a reagent for detecting a cancer according to any one of Claim 12 to 17.

19. The kit for detecting a cancer according to Claim 18, further comprising a standard solution for preparing a calibration curve.

**20.** Use of MDM2, aMDM2 peptide fragment or the labeled products thereof for producing a reagent for detecting a cancer comprising said MDM2, said MDM2 peptide fragment or said labeled products thereof.

**21.** The method according to Claim 8, wherein said anti-MDM2 antibody is either an antibody produced by using a MDM2 peptide fragment as an antigen, or the modified product thereof.

Fig. 1

Measurement result of MDM2 autoantibody concentration by the competition assay

| Disease | Positive rate | Measurement value (%) |
|---|---|---|
| Normal subjct | | |
| Large intestinal cancer | 3/8(37.5%) | |
| Gastric cancer | 7/8(87.5%) | |
| Esophageal cancer | 4/4(100%) | |
| Lung cancer | 8/8(100%) | |
| Pancreatic cancer | 4/4(100%) | |
| Hepatoma | 5/5(100%) | |
| Gallbladder cancer | 2/2(100%) | |
| Prostatic cancer | 1/1(100%) | |
| Breast cancer | 4/8(50.0%) | |

0.0  5.0  10.0  15.0  20.0

(%)

Fig. 2

Calibration curve

Fig. 3

Measurement result of MDM2 autoantibody concentration by the competition assay

| Diagnosis | Number of case | positive rate | MDM2 antibody concentration in serum ($\mu$ g/ml) |
|---|---|---|---|
| Normal subject male | 24 | 0 | |
| female | 25 | 0 | |
| Breast cancer | 36 | 63.9 | |
| Gastric cancer | 51 | 33.3 | |
| Lung cancer | 46 | 65.2 | |

EP 1 271 152 A1

EP 1 271 152 A1

Fig. 4

Measurement result of MDM2 autoantibody in blood by stage in breast cancer

| Stage | Numbers of case | Positive rate % | MDM2 antibody concentration in serum ($\mu$g/ml) |
|---|---|---|---|
| 0 | 1 | 100 | |
| I | 10 | 80.0 | |
| II | 17 | 58.8 | |
| III | 8 | 50.0 | |
| Total | 36 | 63.9 | |

Fig. 5

Measurement result of MDM2 autoantibody in blood by stage in gastric cancer

| Stage | Numbers of case | Positive rate % | MDM2 antibody concentration in serum ($\mu$ g/ml) |
|---|---|---|---|
| I A | 35 | 34.3 | |
| I B | 9 | 44.4 | |
| II | 4 | 25.0 | |
| III | 3 | 0 | |
| Total | 51 | 33.3 | |

EP 1 271 152 A1

Fig. 6

Measurement result of MDM2 autoantibody in blood by histological type in lung cancer

| Histological type | Number of case | Positive rate % | MDM2 antibody concentration in serum ($\mu$g/ml) |
|---|---|---|---|
| Squamous cell carcinoma | 12 | 75.0 | |
| adenocarcinoma | 30 | 60.0 | |
| Large cell carcinlma | 1 | 0 | |
| Small cell carcinoma | 2 | 100 | |
| The other | 1 | 100 | |
| Total | 46 | 65.2 | |

EP 1 271 152 A1

Fig. 7

Measurement result of MDM2 autoantibody in blood by differentiation level in pulmonary squamous cell carcinoma and adenocarcinma

| Histological type and Differentiation level | Number of case | positive rate % | MDM2 antibody concentration in serum ($\mu$ g/ml) |
|---|---|---|---|
| Squamous cell carcinoma Well differentiated type | 1 | 100 | |
| Moderately differentiated type | 7 | 100 | |
| Poorly differentiated type | 4 | 25 | |
| Adenocarcinoma Well differentiated type | 6 | 83 | |
| Moderately differentiated type | 15 | 47 | |
| Poorly differentiated type | 1 | 0 | |

EP 1 271 152 A1

Fig. 8

Measurement result of MDM2 autoantibody in blood by differentiation level in gastric cancer

| Tissue type | Number of case | positive rate % | MDM2 autoantibody concentration in serum ($\mu$ g/ml) | | | | | | | | | |
|---|---|---|---|---|---|---|---|---|---|---|---|---|
| | | | 1 | 2 | 3 | 4 | 5 | 6 | 7 | 8 | 9 | 10 |
| Differentiated gastric cancer | 30 | 36.7 | | | | | | | | | | |
| Undifferentiated gastric cancer | 21 | 28.6 | | | | | | | | | | |
| Total | 51 | 33.3 | | | | | | | | | | |

EP 1 271 152 A1

## INTERNATIONAL SEARCH REPORT

| International application No. |
| --- |
| PCT/JP01/02723 |

**A. CLASSIFICATION OF SUBJECT MATTER**
Int.Cl⁷ G01N 33/574, G01N 33/53, C07K 7/08

According to International Patent Classification (IPC) or to both national classification and IPC

**B. FIELDS SEARCHED**

Minimum documentation searched (classification system followed by classification symbols)
Int.Cl⁷ G01N 33/574, G01N 33/53, C07K 7/08

Documentation searched other than minimum documentation to the extent that such documents are included in the fields searched

Electronic data base consulted during the international search (name of data base and, where practicable, search terms used)
Geneseq/PIR/swiss-prot , BIOSIS, WPI

**C. DOCUMENTS CONSIDERED TO BE RELEVANT**

| Category* | Citation of document, with indication, where appropriate, of the relevant passages | Relevant to claim No. |
| --- | --- | --- |
| X | WO, 95/14233, A (Deutsches Krebsforschungszentrum Stiftung Des Offentlichen Rechts), 20 May, 1997 (20.05.97) & JP, 9-505144, A & EP, 729580, A | 1-21 |
| X | DE, 4339533, A (Deutsches Krebsforschungszentrum Stiftung Des Offentlichen Rechts), 14 June, 1995 (14.06.95) (Family: none) | 1-21 |
| A | Cancer letters, Vol.96, pp.111-115 (1995) | 1-21 |
| A | Nature, Vol.358, pp.80-83 (1992) | 1-21 |

☐ Further documents are listed in the continuation of Box C.  ☐ See patent family annex.

| | |
| --- | --- |
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>08 May, 2001 (08.05.01) | Date of mailing of the international search report<br>22 May, 2001 (22.05.01) |
| --- | --- |
| Name and mailing address of the ISA/<br>Japanese Patent Office | Authorized officer |
| Facsimile No. | Telephone No. |

Form PCT/ISA/210 (second sheet) (July 1992)

## SEQUENCE LISTING

<110> NIPPON KAYAKU KABUSHIKI KAISHA

<120> Detection of cancer by measurement of autoantibody to MDM2, and the reagent thereof

<130> KJ57656A

<150> JP 2000-93569
<151> 2000-03-30

<160> 1

<210> 1
<211> 20
<212> PRT
<213> Homo sapiens

<400> 1

Asn Thr Asn Met Ser Val Pro Thr Asp Gly Ala Val Thr Thr Ser Gln
1               5                   10                  15

Ile Pro Ala Ser
            20